(19) Europäisches Patentamt
European Patent Office
Office européen des brevets

(11) Veröffentlichungsnummer: **0 085 842**
**B1**

# (12) EUROPÄISCHE PATENTSCHRIFT

(45) Veröffentlichungstag der Patentschrift: **21.08.85**

(51) Int. Cl.⁴: **A 61 K 31/41,** A 61 K 31/415 // C07D249/08, C07D233/60

(21) Anmeldenummer: **83100259.7**

(22) Anmeldetag: **14.01.83**

(54) **Antimykotische Mittel.**

(30) Priorität: **27.01.82 DE 3202613**

(43) Veröffentlichungstag der Anmeldung: **17.08.83 Patentblatt 83/33**

(45) Bekanntmachung des Hinweises auf die Patenterteilung: **21.08.85 Patentblatt 85/34**

(84) Benannte Vertragsstaaten: **BE CH DE FR GB IT LI LU NL SE**

(56) Entgegenhaltungen:
**EP - A - 0 040 345**
**EP - A - 0 043 419**
**EP - A - 0 052 424**
**EP - A - 0 054 974**
**EP - A - 0 057 357**
**EP - A - 0 061 051**

(73) Patentinhaber: **BAYER AG, Konzernverwaltung RP Patentabteilung, D-5090 Leverkusen 1 Bayerwerk (DE)**

(72) Erfinder: **Holmwood, Graham, Dr., Katernbergerstrasse 184, D-5600 Wuppertal 1 (DE)**
Erfinder: **Regel, Erik, Untere Bergerheide 26, D-5600 Wuppertal 1 (DE)**
Erfinder: **Jäger, Gerhard, Dr., Gellertstrasse 18, D-5090 Leverkusen 1 (DE)**
Erfinder: **Büchel, Karl Heinz, Prof. Dr., Dabringhausener Strasse 42, D-5093 Burscheid (DE)**
Erfinder: **Plempel, Manfred, Dr., Zwengenberger Strasse 3c, D-5657 Haan (DE)**

**EP 0 085 842 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die vorliegende Erfindung betrifft die Verwendung von neuen substituierten 1-Hydroxyalkyl-azolyl-Derivaten als antimikrobielle Mittel, insbesondere als Antimykotika.

Es ist bereits bekannt geworden, daß bestimmte 1-Hydroxyethyl-azolyl-Derivate, wie beispielsweise 1-(4-Chlorphenoxy)-3,3-dimethyl-2-(imidazol-1-yl-methyl)-2-butanol, 1-(2-Methylphenoxy)-3,3-dimethyl-2-(imidazol-1-yl-methyl)- bzw. (1,2,4-triazol-1-yl-methyl)-2-butanol oder 1-(2-Methylphenyl)-4,4-dimethyl-3-(imidazol-1-yl-methyl)-3-pentanol, gute antimykotische Eigenschaften aufweisen (vergleiche EP-A 0 043 419). Gute antimykotische Eigenschaften sind auch von vielen ähnlichen Azolderivaten bekannt (vergleiche EP-A 0 052 424, EP-A 0 040 345, EP-A 0 061 051, EP-A 0 057 357 und EP-A 0 054 974).

Es wurde gefunden, daß die neuen substituierten 1-Hydroxyalkyl-azolyl-Derivate der allgemeinen Formel

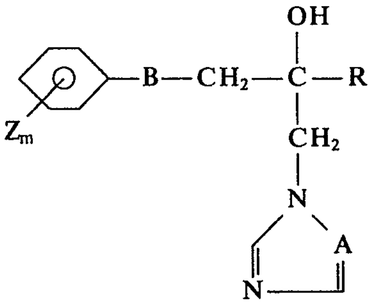

(I)

in welcher

A für ein Stickstoffatom oder die CH-Gruppe steht,
B für Sauerstoff, Schwefel oder die $CH_2$-Gruppe steht,
m für die Zahlen 0, 1, 2 oder 3 steht,
R für die Gruppierungen

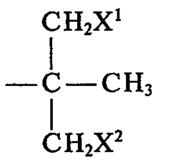 und 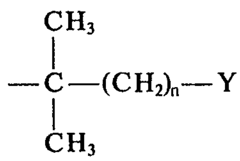

steht, wobei

$X^1$ für Wasserstoff oder Halogen steht;
$X^2$ für Halogen steht;
Y für Alkoxy, Alkylthio, Halogenalkoxy, Halogenalkylthio, Alkenyl mit bis zu 6 Kohlenstoffatomen, Alkoxycarbonyl mit 1 bis 4 Kohlenstoffatomen im Alkylteil, Cyano sowie für gegebenenfalls substituiertes Phenyl, Phenoxy, Phenylthio, Phenylalkoxy mit 1 bis 4 Kohlenstoffatomen im Alkylteil und Phenylalkylthio mit 1 bis 4 Kohlenstoffatomen im Alkylteil steht, wobei jeweils als Phenylsubstituenten vorzugsweise genannt seien: Halogen, Alkyl mit 1 bis 4 Kohlenstoffatomen; Alkoxy und Alkylthio mit jeweils 1 bis 4 Kohlenstoffatomen; Halogenalkyl, Halogenalkoxy und Halogenalkylthio mit jeweils 1 bis 2 Kohlenstoff- und 1 bis 5 gleichen oder verschiedenen Halogenatomen, wie insbesondere Fluor- und Chloratomen, Cyclohexyl, Dialkylamino mit 1 bis 4 Kohlenstoffatomen in jedem Alkylteil, Nitro, Cyano, sowie Alkoxycarbonyl mit 1 bis 4 Kohlenstoffatomen im Alkylteil; und auch für Alkyl mit bis zu 6 Kohlenstoffatomen steht, wenn B für Schwefel steht,
n für die Zahlen 0, 1 oder 2 steht;
Z für Halogen, Alkyl mit 1 bis 4 Kohlenstoffatomen, Cycloalkyl mit 5 bis 7 Kohlenstoffatomen, Alkoxy und Alkylthio mit jeweils 1 bis 4 Kohlenstoffatomen, Halogenalkyl und Halogenalkoxy sowie Halogenalkylthio mit jeweils 1 bis 2 Kohlenstoff- und 1 bis 5 gleichen oder verschiedenen Halogenatomen, wie insbesondere Fluor- und Chloratomen, sowie für jeweils gegebenenfalls durch Halogen und Alkyl mit 1 bis 2 Kohlenstoffatomen substituiertes Phenyl, Phenoxy und Phenylalkyl sowie Phenylalkoxy mit 1 bis 2 Kohlenstoffatomen im Alkylteil bzw. Alkoxyteil steht,

sowie deren Säureadditions-Salze gute antimikrobielle, insbesondere antimykotische Eigenschaften aufweisen.

Die Verbindungen der Formel (I) besitzen ein asymmetrisches Kohlenstoffatom und können deshalb

in den beiden optischen Isomerenformen anfallen.

Überraschenderweise zeigen die erfindungsgemäß zu verwendenden substituierten 1-Hydroxyalkyl-azolyl-Derivate der Formel (I) ein besseres antimykotisches Wirkungsspektrum, insbesondere auch eine bessere, therapeutisch wirksame invivo-Wirksamkeit als die aus dem Stand der Technik bekannten Verbindungen

1-(4-Chlorphenoxy)-3,3-dimethyl-2-(imidazol-1-yl-methyl)-2-butanol,
1-(2-Methylphenoxy)-3,3-dimethyl-2-(imidazol-1-yl-methyl)- bzw.
-(1,2,4-triazol-1-yl-methyl)-2-butanol oder
1-(2-Methylphenyl)-4,4-dimethyl-3-(imidazol-1-yl-methyl)-3-pentanol,

welche chemisch und wirkungsmäßig naheliegende Verbindungen sind. Die erfindungsgemäße Verwendung der Stoffe der Formel (I) stellt somit eine Bereicherung der Pharmazie dar.

Bevorzugt sind diejenigen Verbindungen der Formel (I), in denen

R für die Gruppierungen

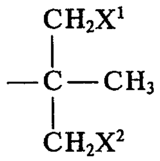

und

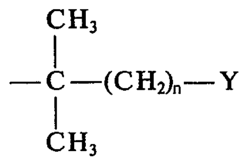

steht, wobei

| | |
|---|---|
| $X^1$ | für Wasserstoff, Fluor, Chlor oder Brom steht, |
| $X^2$ | für Fluor, Chlor oder Brom steht, |
| Y | für Methoxy, Ethoxy, Methylthio, Ethylthio, Trifluormethoxy, Trifluormethylthio, Vinyl, Methoxycarbonyl, Ethoxycarbonyl, Cyano sowie für gegebenenfalls substituiertes Phenyl, Phenoxy, Phenylthio, Phenylmethoxy und Phenylmethylthio steht, wobei jeweils als Phenylsubstituenten genannt seien: Fluor, Chlor, Methyl, Ethyl, Methoxy, Methylthio, Trifluormethyl, Trifluormethoxy, Trifluormethylthio, Dimethylamino, Methoxycarbonyl und Ethoxycarbonyl; und auch für Methyl, Ethyl und Propyl steht, wenn B für Schwefel steht, |
| n | für die Zahlen 0, 1 oder 2 steht, |
| Z | für Fluor, Chlor, Brom, Methyl, tert.-Butyl, Cyclohexyl, Methoxy, Methylthio, Trifluormethyl, Trifluormethoxy, Trifluormethylthio sowie für gegebenenfalls durch Fluor, Chlor und Methyl substituiertes Phenyl, Phenoxy, Benzyl oder Benzyloxy steht, |
| A, B und m | für die in der Erfindungsdefinition angegebenen Bedeutungen stehen. |

Bevorzugte erfindungsgemäße Verbindungen sich auch Additionsprodukte aus Säuren und denjenigen substituierten 1-Hydroxyalkyl-azolyl-Derivaten der Formel (I), in denen die Substituenten A, B, R und $Z_m$ die Bedeutungen haben, die bereits vorzugsweise für diese Substituenten genannt wurden.

Zu den Säuren, die addiert werden können, gehören vorzugsweise Halogenwasserstoffsäuren, wie z. B. die Chlorwasserstoffsäure und die Bromwasserstoffsäure, ferner Phosphorsäure, Salpetersäure, mono- und bifunktionelle Carbonsäuren und Hydroxycarbonsäuren, wie z. B. Essigsäure, Zitronensäure, Maleinsäure, Bernsteinsäure, Fumarsäure, Weinsäure, Zitronensäure, Salizylsäure, Sorbinsäure und Milchsäure, sowie Sulfonsäuren, wie z. B. p-Toluolsulfonsäure und 1,5-Naphthalindisulfonsäure.

Die erfindungsgemäß zu verwendenden substituierten 1-Hydroxyalkyl-azolyl-Derivate und deren Säureadditions-Salze sind noch nicht bekannt. Sie können jedoch gemäß einem eigenen Vorschlag hergestellt werden, indem man

a) Oxirane der Formel

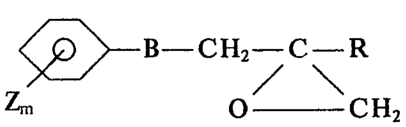

(II)

in welcher

B, R, Z und m die oben angegebene Bedeutung haben,

mit Azolen der Formel

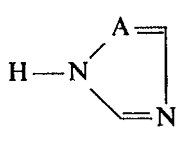

(III)

in welcher

A die oben angegebene Bedeutung hat,

in Gegenwart eines inerten organischen Lösungsmittels, wie beispielsweise eines Alkohols, und gegebenenfalls in Gegenwart einer Base, wie beispielsweise Natriumalkoholat, gegebenenfalls unter einem Druck von 1 bis 25 bar, bei Temperaturen zwischen 60 und 150°C umsetzt; oder

b) Azolylmethyl-oxirane der Formel

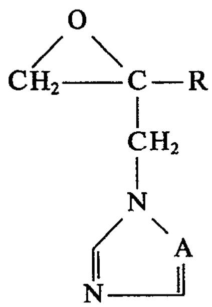

(IV)

in welcher

A und R die oben angegebene Bedeutung haben,

mit (Thio)Phenolen der Formel

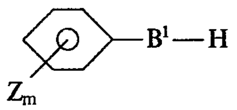

(V)

in welcher

Z und m die oben angegebene Bedeutung haben und
$B^1$ für Sauerstoff oder Schwefel steht,

unter den Bedingungen der Verfahrensvariante (a) umsetzt; und gegebenenfalls an die nach den Verfahrensvarianten (a) und (b) erhaltenen Verbindungen der Formel (I) eine Säure addiert.

Die Säureadditions-Salze der Verbindungen der Formel (I) können in einfacher Weise nach üblichen Salzbildungsmethoden, z. B. durch Lösen einer Verbindung der Formel (I) in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Die Oxirane der Formel (II) sind noch nicht bekannt. Sie können jedoch in allgemein bekannter Art und Weise erhalten werden, indem man Ketone der Formel

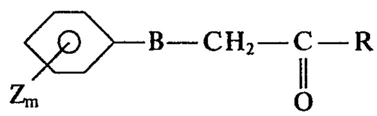

(VI)

in welcher

B, R, Z und m die oben angegebene Bedeutung haben,

entweder

*α*) mit Dimethyloxosulfonium-methylid der Formel

$$(CH_3)_2\overset{\delta^+}{S}\overset{\delta^-}{O}CH_2 \qquad \text{(VII)}$$

in an sich bekannter Weise in Gegenwart eines Verdünnungsmittels, wie beispielsweise Dimethylsulfoxid, bei Temperaturen zwischen 20 und 80°C umsetzt (vergleiche hierzu die Angaben in J. Am. Chem. Soc. 87, 1363—1364 [1965]), oder

β) mit Trimethylsulfonium-methylsulfat der Formel

$$\left[(CH_3)_3\overset{(+)}{S}\right] CH_3\overset{(-)}{SO_4} \qquad (VIII)$$

in an sich bekannter Weise in Gegenwart eines inerten organischen Lösungsmittels, wie z. B. Acetonitril, und in Gegenwart einer Base, wie z. B. Natriummethylat, bei Temperaturen zwischen 0 bis 60°C, vorzugsweise bei Raumtemperatur, umsetzt (vergleiche auch die Angaben in Heterocycles 8, 397 [1977]).

Die so erhaltenen Oxirane der Formel (II) können gegebenenfalls ohne Isolierung direkt weiter umgesetzt werden.

Die bei der Herstellung der Oxirane der Formel (II) als Ausgangsstoffe benötigten Ketone der Formel (VI) sind bekannt (vergleiche z. B. DE-OS 2 632 603, DE-OS 2 632 602, DE-OS 2 635 664, DE-OS 2 635 666, DE-OS 2 918 894, DE-OS 2 918 893, DE-OS 2 201 063, DE-OS 2 705 678, DE-OS 2 737 489), bzw. sind sie Gegenstand eigener älterer Patentanmeldungen, die noch nicht veröffentlicht sind (vergleiche die Deutschen Patentanmeldungen P 3 021 551 vom 07. 06. 1980, P 3 119 390 vom 15. 05. 1981, P 3 101 143 vom 16. 01. 1981), bzw. lassen sie sich nach im Prinzip bekannten Verfahren herstellen.

Das bei der Verfahrensvariante (α) benötigte Dimethyloxosulfoniummethylid der Formel (VII) ist ebenfalls bekannt (vergleiche J. Amer. Chem. Soc. 87, 1363–1364 [1965]). Es wird bei der obigen Umsetzung in frisch zubereitetem Zustand verarbeitet, indem man es in situ durch Umsetzung von Trimethyloxosulfoniumiodid mit Natriumhydrid bzw. Natriumamid in Gegenwart eines Verdünnungsmittels erzeugt.

Das bei der Verfahrensvariante (β) benötigte Trimethylsulfonium-methylsulfat der Formel (VIII) ist ebenfalls bekannt (vgl. Heterocycles 8, [1977]). Es wird bei der obigen Umsetzung ebenfalls in frisch hergestelltem Zustand eingesetzt, indem man es durch Reaktion von Dimethylsulfid mit Dimethylsulfat in situ erzeugt.

Die Azole der Formel (II) sind allgemein bekannte Verbindungen der organischen Chemie.

Die Azolylmethyl-oxirane der Formel (IV) sind noch nicht bekannt. Sie können in allgemein bekannter Art und Weise erhalten werden, indem man Azolo-ketone der Formel

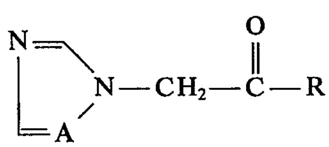

(IX)

in welcher

A und R die oben angegebene Bedeutung haben,

entsprechend den oben angegebenen Verfahrensvarianten (α) und (β) epoxidiert.

Die Azolo-ketone der Formel (IX) sind bekannt (vergleiche DE-OS 2 431 407, DE-OS 2 638 470, DE-OS 2 820 361) bzw. lassen sie sich nach im Prinzip bekannten Verfahren herstellen.

Die (Thio)Phenole der Formel (V) sind allgemein bekannte Verbindungen der organischen Chemie.

Die erfindungsgemäß verwendbaren Verbindungen der Formel (I) und ihre Säureadditions-Salze weisen antimikrobielle, insbesondere starke antimykotische Wirkungen auf. Sie besitzen ein sehr breites antimykotisches Wirkungsspektrum, insbesondere gegen Dermatophyten und Sproßpilze sowie biphasische Pilze, z. B. gegen Candida-Arten, wie Candida albicans, Epidermophyton-Arten, wie Epidermophyton floccosum, Aspergillus-Arten, wie Aspergillus niger und Aspergillus fumigatus, Trichophyton-Arten, wie Trichophyton mentagrophytes, Microsporon-Arten, wie Microsporon felineum sowie Torulopsis-Arten, wie Torulopsis glabrata. Die Aufzählung dieser Mikroorganismen stellt keinesfalls eine Beschränkung der bekämpfbaren Keime dar, sondern hat nur erläuternden Charakter.

Als Indikationsgebiete in der Humanmedizin können beispielsweise genannt werden:

Dermatomykosen und Systemmykosen durch Trichophyton mentagrophytes und andere Trichophytonarten, Microsporonarten, Epidermophyton floccosum, Sproßpilze und bi-phasische Pilze sowie Schimmelpilze hervorgerufen.

Als Indikationsgebiete in der Tiermedizin können beispielsweise aufgeführt werden:

Alle Dermatomykosen und Systemmykosen, insbesondere solche, die durch die obengenannten Erreger hervorgerufen werden.

Zur vorliegenden Erfindung gehören pharmazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoffen bestehen sowie Verfahren zur Herstellung dieser Zubereitungen.

Die vorliegenden Erfindung gehören auch pharmazeutische Zubereitungen in Dosierungseinheiten. Dies bedeutet, daß die Zubereitungen in Form einzelner Teile, z. B. Tabletten, Dragees, Kapseln, Pillen, Suppositorien und Ampullen vorliegen, deren Wirkstoffgehalt einem Bruchteil oder einem Vielfachen einer Einzeldosis entspricht. Die Dosierungseinheiten können z. B. 1, 2, 3 oder 4 Einzeldosen oder ½, ⅓ oder ¼ einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z. B. Stärken, Milchzucker, Rohrzucker, Glucose, Mannit und Kieselsäure, (b) Bindemittel, z. B. Carboxymethylcellulose, Alginate, Gelatine, Polyvinylpyrrolidon, (c) Feuchthaltemittel, z. B. Glycerin, (d) Sprengmittel, z. B. Agar-Agar, Calciumcarbonat und Natriumbicarbonat, (e) Lösungsverzögerer, z. B. Paraffin und (f) Resorptionsbeschleuniger, z. B. quaternäre Ammoniumverbindungen, (g) Netzmittel, z. B. Cetylalkohol, Glycerinmonostearat, (h) Adsorptionsmittel, z. B. Kaolin und Bentonit und (i) Gleitmittel, z. B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z. B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z. B. Polyethylenglykole, Fette, z. B. Kakaofett und höhere Ester (z. B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z. B. tierische und pflanzliche Fette, Wachse, Paraffine, Stärke, Tragant, Cellulosederivate, Polyethylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z. B. Milchzucker, Talkum, Kieselsäure, Aluminiumhydroxid, Calciumsilikat und Polyamidpulver oder Gemische dieser Stoffe. Sprays können zusätzlich die üblichen Treibmittel, z. B. Chlorfluorkohlenwasserstoffe enthalten.

Lösungen und Emulsionen können neben dem oder den Wirkstoffen die üblichen Trägerstoffe wie Lösungsmittel, Lösungsvermittler und Emulgatoren, z. B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur patenteralen Applikation können die Lösungen und Emulsionen auch in steriler und blutisotonischer Form vorliegen.

Suspensionen können neben dem oder den Wirkstoffen die üblichen Trägerstoffen wie flüssige Verdünnungsmittel, z. B. Wasser, Ethylalkohol, Propylenglykol, Suspendiermittel, z. B. ethoxylierte Isostearylalkohole, Polyoxyethylensorbit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbessernde Zusätze, z. B. Pfefferminzöl und Eukalyptusöl und Süßmittel, z. B. Saccharin enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können außer den erfindungsgemäßen Wirkstoffen auch weitere pharmazeutischen Wirkstoffe enthalten.

Die Herstellung der oben aufgeführten pharmazeutischen Zubereitungen erfolgt in üblicher Weise nach bekannten Methoden, z. B. durch Mischen des oder der Wirkstoffe mit dem oder den Trägerstoffen.

Die Wirkstoffe oder die pharmazeutischen Zubereitungen können lokal, oral, parenteral, intraperitoneal und/oder rektal, vorzugsweise parenteral, insbesondere intravenös appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 10 bis etwa 300,

vorzugsweise 50 bis 200 mg/kg Körpergewicht je 24 Stunden, gegebenenfalls in Form mehrerer Einzelgaben zur Erzielung der gewünschten Ergebnisse zu verabreichen.

Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

## Herstellungsbeispiele

### Beispiel 1

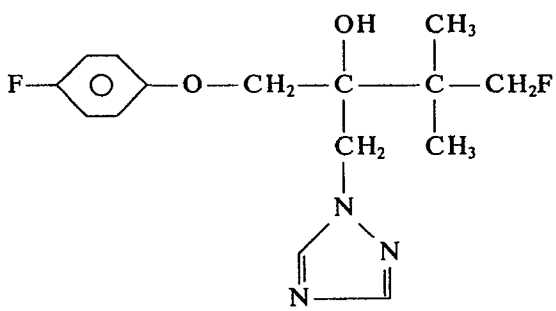

(Verfahren a)

0,46 g (0,02 Mol) Natrium in 200 ml n-Propanol werden mit 15,2 g (0,22 Mol) 1,2,4-Triazol versetzt. Man erhitzt die Lösung zum Sieden und versetzt tropfenweise mit 48,5 g (0,2 Mol) 2-(4-Fluorphenoxymethyl)-2-(fluor-tert.-butyl)-oxiran, gelöst in 50 ml n-Propanol. Man läßt die Reaktionslösung 48 Stunden unter Rückfluß nachrühren, engt ein und versetzt den Rückstand mit 200 ml Essigester und 100 ml Wasser. Die organische Phase wird abgetrennt, zweimal mit Wasser gewaschen, über Natriumsulfat getrocknet und eingeengt. Der Rückstand wird in Ether gelöst und mit Chlorwasserstoff begast. Der entstehende Niederschlag wird abgesaugt, mit Ether gewaschen und mit Essigester/1n Natronlauge versetzt. Das anfallende Rohprodukt wird über eine Kieselgelsäule (Laufmittel: Methylenchlorid/Essigester, 2 : 1) gereinigt und aus Acetonitril umkristallisiert. Man erhält 17,9 g (29% der Theorie) 4-Fluor-2-(4-fluorphenoxymethyl)-3,3-dimethyl-1-(1,2,4-triazol-1-yl)-butan-2-ol vom Schmelzpunkt 97–99°C.

### Herstellung des Ausgangsproduktes

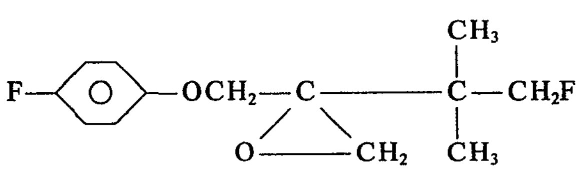

Eine Lösung von 47,3 ml (0,5 Mol) Dimethylsulfat in 250 ml absolutem Acetonitril wird bei Raumtemperatur mit einer Lösung von 40,5 ml (0,55 Mol) Dimethylsulfid in 50 ml absolutem Acetonitril versetzt. Man läßt die Reaktionsmischung 4 Tage bei Raumtemperatur rühren. Danach werden 55,1 g (0,24 Mol) 4-Fluor-1-(4-chlorphenoxy)-3,3-dimethyl-butan-2-on in 50 ml absolutem Acetonitril zugetropft und dann 30,4 g (0,56 Mol) Natriummethylat zugesetzt. Man läßt das Reaktionsgemisch 4 Tage nachrühren. Anschließend wird eingeengt, der Rückstand zwischen Wasser und Essigester verteilt, die organische Phase abgetrennt, zweimal mit Wasser und einmal mit gesättigter Natriumchloridlösung gewaschen, über Natriumsulfat getrocknet, eingeengt und der Rückstand im Vakuum destilliert. Man erhält 49,1 g (85% der Theorie) 2-(4-Fluorphenoxymethyl)-2-(fluor-tert.-butyl)-oxiran vom Siedepunkt 81–83°C/0,09 mbar.

Beispiel 2

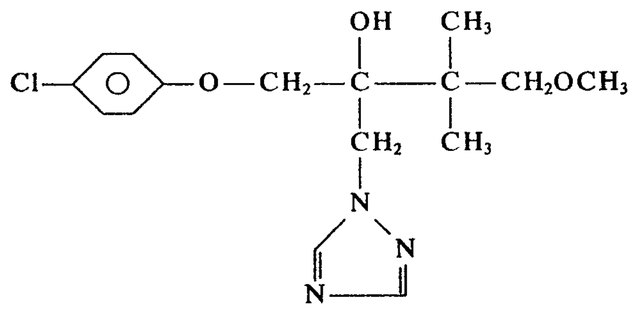

(Verfahren b)

8,1 g (0,0633 Mol) 4-Chlorphenol werden zu einer Lösung von 0,32 g (0,014 Mol) Natrium in 100 ml absolutem Ethanol gegeben. Dazu werden 10,3 g (0,0488 Mol) 2-(Methoxy-tert.-butyl)-2-(1,2,4-triazol-1-yl-methyl)-oxiran, gelöst in 300 ml absolutem Ethanol, gegeben und das Reaktionsgemisch 48 Stunden unter Rückfluß erhitzt. Man läßt abkühlen und engt das Reaktionsgemisch ein. Der Rückstand wird in Essigester aufgenommen, einmal mit Wasser, einmal mit 1 n Natronlauge, erneut mit Wasser, danach mit 1 n Salzsäure und 1 n Natronlauge, wiederum mit Wasser und anschließend mit gesättigter Kochsalzlösung gewaschen. Die organische Phase wird über Natriumsulfat getrocknet und eingeengt. Man erhält 10,7 g (64,4% der Theorie) 2-(2-Chlorphenoxymethyl)-3,3-dimethyl-4-methoxy-1-(1,2,4-triazol-1-yl)-butan-2-ol vom Brechungsindex $n_D^{20} = 1{,}531$.

Herstellung des Ausgangsproduktes

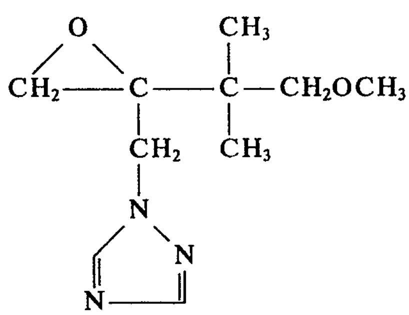

Unter Stickstoff-Atmosphäre werden 24 g (1 Mol) Natriumhydrid (80%ig in Paraffinöl) und 220 g (1 Mol) Trimethylsulfoxoniumiodid bei 10°C gemischt und langsam mit 1000 ml Dimethylsulfoxid versetzt. Nach einer Stunde werden in die Suspension 177,5 g (0,9 Mol) 3,3-Dimethyl-4-methoxy-1-(1,2,4-triazol-1-yl)-butan-2-on, gelöst in 200 ml Tetrahydrofuran, zugetropft.

Das Gemisch wird 2 Tage bei Raumtemperatur gerührt. Die Lösungsmittel werden im Hochvakuum abgezogen, der Rückstand mit Essigester verrührt und abgesaugt. Das Filtrat wird dreimal mit verdünnter Kochsalzlösung gewaschen, über Natriumsulfat getrocknet, eingeengt, und der Rückstand wird im Hochvakuum destilliert. Man erhält 163 g (86% der Theorie) 2-(Methoxy-tert.-butyl)-2-(1,2,4-triazol-1-yl-methyl)-oxiran vom Siedepunkt 89—92°C/0,008 mbar.

Beispiel 3

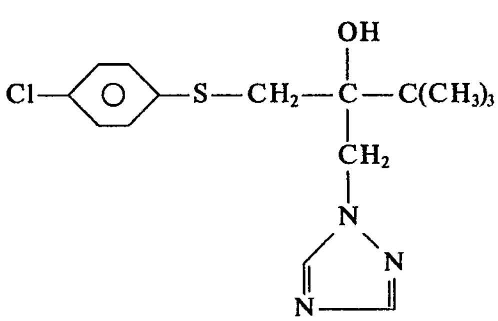

(Verfahren b)

5,4 g (0,1 Mol) Natriummethylat werden portionsweise in ein Gemisch von 18 g (0,1 Mol) 2-tert.-Butyl-2-(1,2,4-triazol-1-yl-methyl)-oxiran und 14,5 g (0,1 Mol) 4-Chlorthiophenol in 250 ml Acetonitril eingetragen. Man läßt 16 Stunden unter Rückfluß rühren und engt im Vakuum ein. Der Rückstand wird in Chloroform gelöst, mit Wasser gewaschen, über Natriumsulfat getrocknet, filtriert und eingeengt. Das verbleibende Öl wird über eine Kieselgelsäule chromatographiert. Man erhält 6,9 g (21% der Theorie) 2-(4-Chlorphenylthiomethyl)-3,3-dimethyl-1-(1,2,4-triazol-1-yl)-butan-2-ol vom Schmelzpunkt 54—55°C.

Herstellung des Ausgangsproduktes

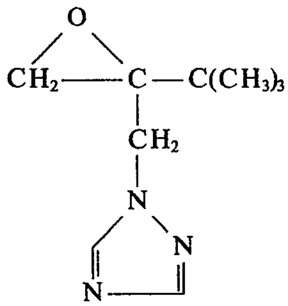

6 g (0,11 Mol) Natriummethylat werden portionsweise in eine Suspension von 24 g (0,11 Mol) Trimethylsulfoxoniumiodid in 24 g Dimethylsulfoxid eingetragen. Man versetzt zusätzlich mit 10 ml Tetrahydrofuran und läßt anschließend das Reaktionsgemisch 5 Stunden bei Raumtemperatur rühren. Danach wird eine Lösung von 16,8 g (0,1 Mol) 1,2,4-Triazol-1-yl-pinakolin in 100 ml Tetrahydrofuran zugetropft und das Gemisch 5 Stunden auf 70°C erhitzt. Das Reaktionsgemisch wird filtriert und das Filtrat im Vakuum eingeengt. Der Rückstand wird in Chloroform gelöst, mit Wasser gewaschen, über Natriumsulfat getrocknet, filtriert und eingeengt. Man erhält 13,8 g 69,4%iges 2-tert.-Butyl-(1,2,4-triazol-1-yl-methyl)-oxiran vom Brechungsindex $n_D^{20} = 1,4872$.

Beispiel 4

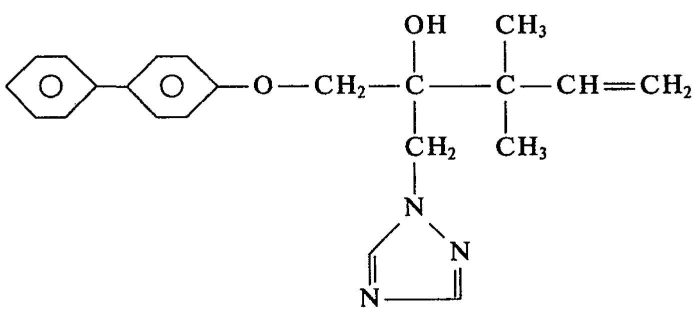

(Verfahren a)

Zu einer siedenden Lösung von 1 g (0,012 Mol) Natriumpropylat und 9,1 g (0,132 Mol) 1,2,4-Triazol in 50 ml n-Propanol werden 35,3 g (0,120 Mol) 2-(4-Biphenylyloxymethyl)-2-(3,3-dimethyl-1-propan-3-yl)-oxiran, gelöst in 80 ml n-Propanol, zugegeben. Nach zweitägigem Refluxieren wird die Lösung unter vermindertem Druck eingedampft, der ölige Rückstand in 200 ml Essigester aufgenommen und dreimal mit je 50 ml Wasser gewaschen. Nach dem Trocknen der organischen Phase über wasserfreiem Natriumsulfat wird die Lösung unter vermindertem Druck eingedampft. Nach Verreiben des verbleibenden Rückstandes mit 150 ml Essigester erhält man 6,6 g (15,1% der Theorie) 4-(4-Biphenylyl oxymethyl)-3,3-dimethyl-4-hydroxy-5-(1,2,4-triazol-4-yl)-1-penten als farblose Kristalle vom Schmelzpunkt 171—173°C. Das nach dem Eindampfen des Filtrats verbleibende Öl (37,1 g) wird in 200 ml Aceton aufgenommen und mit einer Lösung von 34,6 g Naphthalindisulfonsäure-(1,5) in 100 ml Aceton versetzt. Das auskristallisierende Salz wird abgenutscht, mit Aceton gewaschen und in einem Gemisch von je 250 ml Wasser und Dichlormethan aufgenommen. Durch Einrühren von 10%iger Natriumcarbonat-Lösung wird alkalisch gestellt. Durch Eindampfen der organischen Phase erhält man 24 g eines Öls, das in Essigester gelöst und anschließend über Kieselgel filtriert wird. Nach

Eindampfen des Filtrats erhält man 17,8 g (40,8% der Theorie) 4-(4-Biphenylyloxymethyl)-3,3-dimethyl-4-hydroxy-5-(1,2,4-triazol-4-yl)-1-penten in Form farbloser Kristalle vom Schmelzpunkt 85–87°C.

Herstellung des Ausgangsproduktes

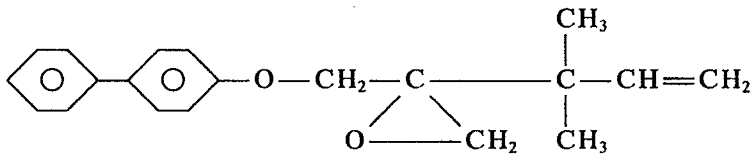

Zu einer Lösung von 49,4 ml (0,518 Mol) Dimethylsulfat in 250 ml Acetonitril läßt man unter Außenkühlung bei 25°C im Laufe von 2 Stunden eine Lösung von 41,9 ml (0,567 Mol) Dimethylsulfid in 50 ml Acetonitril zutropfen. Nach viertägigem Stehen bei Raumtemperatur werden dann innerhalb 1 Stunde 87,4 g (0,31 Mol) 5-(4-Biphenylyloxy)-3,3-dimethyl-1-penten-4-on, gelöst in 90 ml Acetonitril, eingerührt. Anschließend werden bei 25°C 31,6 g (0,58 Mol) Natriummethylat eingetragen. Nach zwölfstündigem Rühren bei Raumtemperatur wird vom ausgeschiedenen Salz abfiltriert und das Filtrat eingedampft. Der ölige Rückstand wird in 500 ml Essigester aufgenommen, die Lösung zweimal mit je 500 ml Wasser gewaschen und über wasserfreiem Natriumsulfat getrocknet. Nach dem Eindampfen im Vakuum werden 73,4 g (80,4% der Theorie) 2-(4-Bisphenylyloxymethyl)-2-(3,3-dimethyl-1-propen-3-yl)-oxiran als farblose, wachsartige Masse erhalten.

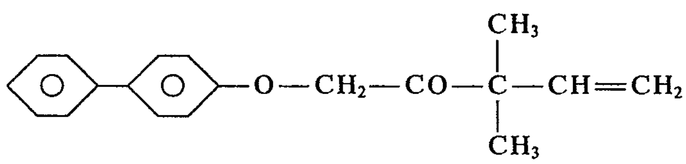

Zu einem Gemisch aus 184,9 g (1,34 Mol) Kaliumcarbonat und 227,8 g (1,34 Mol) 4-Hydroxybiphenyl in 1000 ml siedendem Aceton werden innerhalb einer Stunde 196,5 g (1,34 Mol) 5-Chlor-3,3-dimethyl-1-penten-4-on zugetropft. Man erhitzt nach 5 Stunden zum Sieden, läßt das Gemisch auf Raumtemperatur abkühlen und filtriert vom ausgeschiedenen Kaliumchlorid ab. Das nach dem Eindampfen des Filtrats verbleibende Öl wird in 1000 ml Essigester aufgenommen. Man wäscht zweimal mit je 150 ml 10%iger Natriumcarbonat-Lösung und Wasser, trocknet über wasserfreiem Natriumsulfat und destilliert das Lösungsmittel ab. Restmengen an Lösungsmittel werden bei 0,1 Torr (Badtemperatur: 180°C) abgezogen. Man erhält so 357,2 g (95% der Theorie) 5-(4-Biphenylyloxy)-3,3-dimethyl-1-penten-4-on vom Schmelzpunkt 42–44°C.

Beispiel 5

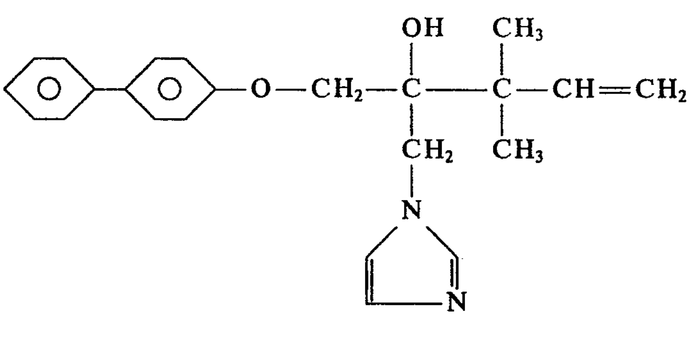

(Verfahren a)

Zu einer siedenden Lösung von 1 g (0,012 Mol) Natriumpropylat und 9 g (0,132 Mol) Imidazol in 50 ml n-Propanol werden innerhalb von 5 Minuten 35,3 g (0,120 Mol) 2-(4-Biphenylyloxymethyl)-2-(3,3-dimethyl-1-propen-3-yl)-oxiran, gelöst in 80 ml n-Propanol, zugegeben. Nach zweitägigem Refluxieren der Lösung wird das Lösungsmittel unter vermindertem Druck abdestilliert, der Rückstand in einem Gemisch aus 150 ml Essigester und 100 ml Dichlormethan aufgenommen und dreimal mit je 50 ml Wasser gewaschen. Nach dem Trocknen der organischen Phase über wasserfreiem Natriumsulfat wird die Lösung unter vermindertem Druck zur Trockne eingedampft. Der verbleibende feste Rückstand wird mit 10 ml Essigester versetzt und abfiltriert. Man erhält so 27,2 g (62,5% der Theorie)

4-(4-Biphenylyloxymethyl)-3,3-dimethyl-4-hydroxy-5-(imidazol-1-yl)-1-penten in Form farbloser Kristalle vom Schmelzpunkt 151–153°C.

In analoger Weise und entsprechend den erfindungsgemäßen Verfahren (a) und (b) werden die nachfolgenden Verbindungen der allgemeinen Formel

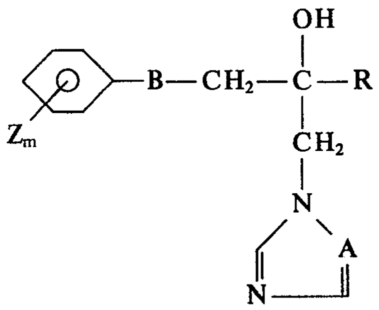

(I)

erhalten:

| Beispiel Nr. | $Z_m$ | B | A | R | Schmelzpunkt (°C) bzw. Brechungsindex $n_D^{20}$ |
|---|---|---|---|---|---|
| 6 | 4-$CH_3$ | O | N | $-C(CH_3)_2CH_2F$ | 91-92 |
| 7 | 4-Cl | O | N | $-C(CH_3)_2CH_2F$ | 111-113 |
| 8 | 4-Cl, 2-$CH_3$ | O | N | $-C(CH_3)_2CH_2F$ | 119-120 |
| 9 | 2,4-$Cl_2$ | O | N | $-C(CH_3)_2CH_2F$ | 107-109 |
| 10 | 4-Cl | S | N | $-C(CH_3)_2CH_2F$ | 87 |
| 11 | 2-Cl | S | N | $-C(CH_3)_2CH_2F$ | 1,552 |
| 12 | 3,4-$Cl_2$ | S | N | $-C(CH_3)_2CH_2F$ | 1,5710 |
| 13 | 4-Cl | O | N | $-C(CH_2F)_2CH_3$ | 111-112 |
| 14 | 2,4-$Cl_2$ | O | N | $-C(CH_2F)_2CH_3$ | 109-110 |
| 15 | 4-Cl, 2-$CH_3$ | O | N | $-C(CH_2F)_2CH_3$ | 138-139 |
| 16 | 2,4-$Cl_2$ | O | N | $-C(CH_3)_2CH_2OCH_3$ | 49-55 |
| 17 | 4-Cl, 2-$CH_3$ | O | N | $-C(CH_3)_2CH_2OCH_3$ | 1,537 |
| 18 | 4-Cl | O | N | $-C(CH_3)_2CH_2OC_2H_5$ | 69-73 |
| 19 | 4-Cl | O | N | $-C(CH_3)_2CH_2O-$ 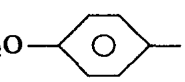 $-Cl$ | 125-126 |
| 20 | 4-F | O | N | $-C(CH_3)_2CH_2OCH_3$ | 1,5152 |
| 21 | 2,4-$Cl_2$ | O | N | $-C(CH_3)_2-$ 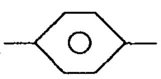 $-Cl$ | 1,5805 |
| 22 | 4-Cl | O | N | $-C(CH_3)_2-CH=CH_2$ | 58-59 |
| 23 | 2,4-$Cl_2$ | O | N | $-C(CH_3)_2-CH=CH_2$ | 86-87 |
| 24 | 4-F | O | N | $-C(CH_3)_2-CH=CH_2$ | 66-68 |
| 25 | 4-Cl | $CH_2$ | N | $-C(CH_3)_2CH_2F$ | 110-112 |
| 26 | 2,4-$Cl_2$ | $CH_2$ | N | $-C(CH_3)_2CH_2F$ | 88-90 |

Fortsetzung

| Beispiel Nr. | $Z_m$ | B | A | R | Schmelzpunkt (°C) bzw. Brechungsindex $n_D^{20}$ |
|---|---|---|---|---|---|
| 27 | 2,4-$Cl_2$ | $CH_2$ | N | $-C(CH_2F)_2CH_3$ | 97–99 |
| 28 | 4-Cl | $CH_2$ | N | $-C(CH_2F)_2CH_3$ | 122–124 |
| 29 | 4-Cl | $CH_2$ | N | $-C(CH_3)_2CH_2O-$ 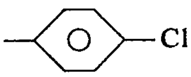 | 120–121 |
| 30 | 4-Cl | $CH_2$ | N | $-C(CH_3)_2O-$ 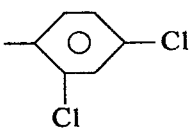 | 116–118 |
| 31 | 4-Cl | O | CH | $-C(CH_2F)_2CH_3$ | 165–167 |
| 32 | 2,4-$Cl_2$ | O | CH | $-C(CH_2F)_2CH_3$ | 148–150 |
| 33 | 4-Cl, 2-$CH_3$ | O | CH | $-C(CH_2F)_2CH_3$ | 133–135 |
| 34 | 4-Cl | $CH_2$ | CH | $-C(CH_3)_2O-$ 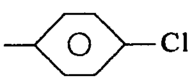 | 118–120 |
| 35 | 4-Cl | $CH_2$ | CH | $-C(CH_3)_2CH_2O-$ 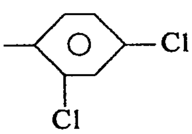 | 121–123 |
| 36 | 2,4-$Cl_2$ | $CH_2$ | CH | $-C(CH_2F)_2CH_3$ | 96–99 |
| 37 | 4-Cl | $CH_2$ | CH | $-C(CH_2F)_2CH_3$ | 128–137 |
| 38 | 2,4-Cl | $CH_2$ | CH | $-C(CH_3)_2CH_2F$ | 103–106 |
| 39 | 2,4-Cl | O | CH | $-C(CH_3)_2-CH=CH_2$ | 136–137 |
| 40 | 4-F | O | CH | $-C(CH_3)_2-CH=CH_2$ | 135–136 |
| 41 | 3,4-$Cl_2$ | S | N | $-C(CH_3)_3$ | 97 |
| 42 | 4-$OCF_3$ | O | N | $-C(CH_3)_2CH_2OCH_3$ | 1,4882 |
| 43 | 4-$OCF_3$ | O | N | $-C(CH_3)_2CH_2OC_2H_5$ | 1,4836 |
| 44 | 4-Cl | O | N | $-C(CH_3)_2-O-$ 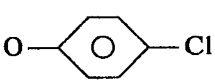 | Harz |
| 45 | 4-F | O | N | $-C(CH_3)_2-O-$ 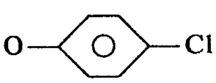 | Harz |
| 46 | 2,4-Cl | O | N | $-C(CH_3)_2-O-$ 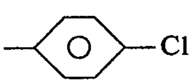 | Harz |
| 47 | 4- | O | N | $-C(CH_3)_2-O-$ 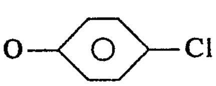 | Harz |
| 48 | 4-Cl | O | N | $-C(CH_3)_2-O-$ 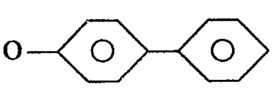 | Harz |
| 49 | 2,4-$Cl_2$ | O | N | $-C(CH_3)_2-O-$ 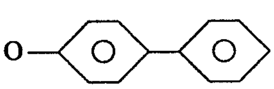 | Harz |

Fortsetzung

| Beispiel Nr. | $Z_m$ | B | A | R | Schmelzpunkt (°C) bzw. Brechungsindex $n_D^{20}$ |
|---|---|---|---|---|---|
| 50 | 2,4-$Cl_2$ | O | N | 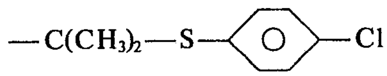 | 115 |
| 51 | 4-Cl | O | N | 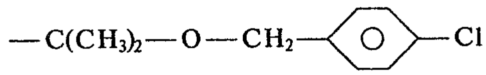 | 133 |
| 52 | 2,4-$Cl_2$ | O | N | 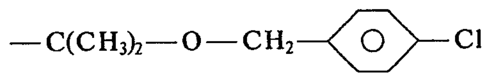 | 102 |

Verwendungsbeispiele

In den nachfolgenden Beispielen werden die aus DE-OS 3 018 865 bekannten Verbindungen (A) bis (D) als Vergleichssubstanzen eingesetzt:

(A) 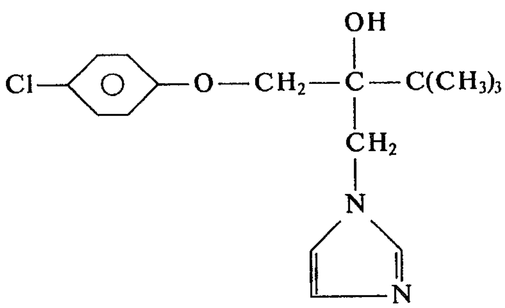

(B) 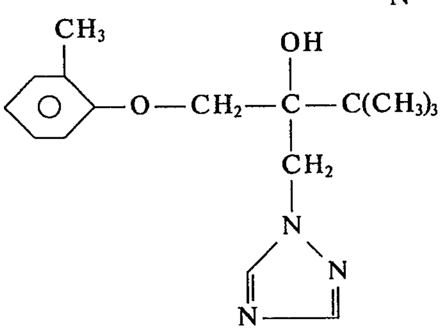

(C) 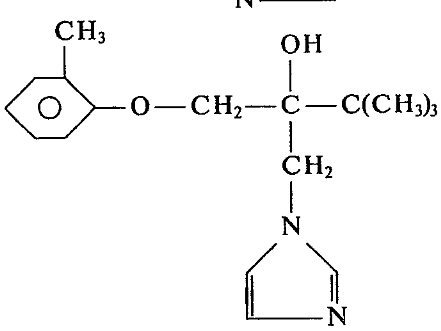

(D) 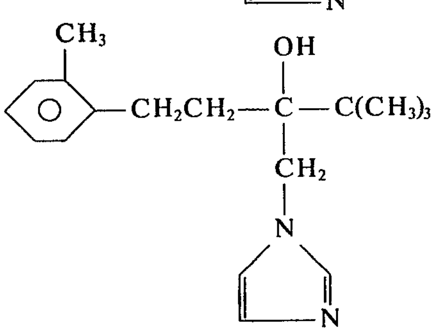

## Beispiel A

### Antimykotische in-vitro-Wirksamkeit

Versuchsbeschreibung:

Die in-vitro-Prüfungen wurden im Reihenverdünnungstest mit Keiminokula von durchschnittlich $5 \times 10^4$ Keimen/ml Substrat durchgeführt. Als Nährmittel dienten

a) für Dermatophyten und Schimmelpilze: Sabouraud's milieu d'épreuve
b) für Hefen: Fleischextrakt-Traubenzucker-Bouillon.

Die Bebrütungstemperatur betrug 20° C, die Bebrütungsdauer lag bei 24 bis 96 Stunden bei Hefen und 96 Stunden bei Dermatophyten und Schimmelpilzen.
In diesem Test zeigen die erfindungsgemäßen Verbindungen der Formel (I) eine bessere antimykotische Wirkung als die aus dem Stand der Technik bekannten Verbindungen (A), (B), (C) und (D).

Tabelle A

Antimykotische in-vitro-Wirksamkeit

| Wirkstoff | MHK-Werte in γ/ml Nährmedium | | | | |
|---|---|---|---|---|---|
| | Tricho-phyton mentagr. | Micro-sporum canis | Candida albicans | Toru-lopsis glabrata | Asper-gillus fumigatus |
| (A) (bekannt) | 8 | 16 | <1 | 2 | 32 |
| (B) (bekannt) | 4 | 4 | 32 | >64 | 32 |
| (C) (bekannt) | 32 | – | 32 | >64 | >64 |
| (D) (bekannt) | 8 | 16 | 2 | 16 | 64 |
| Verbindungen gemäß Herstellungs-Beispiel | | | | | |
| 1 | 4 | 8 | <1 | 16 | 8 |
| 2 | 2 | 4 | <1 | 8 | 8 |
| 3 | <1 | <1 | <1 | 2 | <1 |
| 4 | <1 | <1 | <1 | 32 | <1 |
| 5 | <1 | <1 | <1 | <1 | <1 |
| 6 | 2 | 4 | <1 | 4 | 2 |
| 7 | <1 | 2 | <1 | <1 | <1 |
| 8 | <1 | <1 | <1 | 2 | 4 |
| 9 | <1 | 2 | 4 | 4 | 8 |
| 10 | <1 | <1 | 4 | 16 | <1 |
| 11 | 2 | 4 | <1 | 16 | 8 |
| 12 | <1 | 2 | <1 | <1 | <1 |

Fortsetzung

| Wirkstoff | MHK-Werte in γ/ml Nährmedium | | | | |
|---|---|---|---|---|---|
| | Tricho-phyton mentagr. | Micro-sporum canis | Candida albicans | Toru-lopsis glabrata | Asper-gillus fumigatus |
| Verbindungen gemäß Herstellungs-Beispiel | | | | | |
| 13 | <1 | 2 | <1 | 8 | 4 |
| 14 | <1 | <1 | <1 | 4 | 4 |
| 15 | <1 | <1 | <1 | 4 | 8 |
| 16 | <1 | 2 | <1 | 2 | 8 |
| 17 | <1 | <1 | <1 | 4 | 4 |
| 18 | <1 | 4 | <1 | 4 | 4 |
| 19 | <1 | 2 | 8 | 2 | <1 |
| 20 | 2 | 8 | <1 | 2 | 32 |
| 22 | <1 | 4 | 2 | 16 | <1 |
| 23 | <1 | 8 | <1 | 4 | 4 |
| 24 | <1 | 8 | 4 | 8 | 4 |
| 25 | <1 | <1 | <1 | 2 | <1 |
| 26 | <1 | 2 | 4 | 8 | <1 |
| 27 | <1 | 2 | 2 | 4 | 2 |
| 28 | <1 | 2 | 2 | 2 | <1 |
| 29 | <1 | 2 | 16 | 2 | <1 |
| 30 | <1 | 64 | 16 | 8 | 4 |
| 32 | 2 | 16 | 2 | 8 | 32 |
| 34 | <1 | <1 | <1 | <1 | <1 |
| 35 | <1 | <1 | 4 | <1 | <1 |
| 36 | 2 | 4 | <1 | 4 | 8 |
| 37 | 4 | 16 | 4 | 2 | 2 |
| 38 | 4 | 8 | <1 | 2 | 16 |
| 39 | <1 | 16 | 2 | <1 | 32 |

Beispiel B

Antimykotische in-vitro-Wirksamkeit (oral) bei Mäusecandidose

Versuchsbeschreibung:

Mäuse vom Typ SPF-$CF_1$ wurden intravenös 1–2 × $10^6$ logarithmisch wachsenden Candida-Zellen, die in physiologischer Kochsalzlösung suspendiert werden, infiziert. Eine Stunde vor und sieben Stunden nach der Infektion werden die Tiere mit jeweils 50–100 mg/kg Körpergewicht der Präparate oral behandelt.

Ergebnis:

Unbehandelte Tiere starben 3 bis 6 Tage post infektionem. Die Überlebensrate am 6. Tag post infektionem betrug bei unbehandelten Kontrolltieren etwa 5%.
In diesem Test zeigten z. B. die erfindungsgemäßen Verbindungen: 1, 3, 4, 5, 7, 8, 9, 10, 12, 13, 14, 15, 22, 23, 24, 25, 26, 27, 28, 31, 32, 36, 37, 38, 39 und 40 eine bessere Wirkung als die aus dem Stand der Technik bekannten Verbindungen (A), (B), (C) und (D).

Zeichenerklärung:

| | | | | |
|---|---|---|---|---|
| +++++ | = | sehr gute Wirkung | = | 90% Überlebende am 6. Tag p. i. |
| ++++ | = | gute Wirkung | = | 80% Überlebende am 6. Tag p. i. |
| +++ | = | Wirkung | = | 60% Überlebende am 6. Tag p. i. |
| ++ | = | schwache Wirkung | = | 40% Überlebende am 6. Tag p. i. |
| + | = | Spur Wirkung | = | unter 40% Überlebende am 6. Tag p. i. |
| k. W. | = | keine Wirkung | | |

Tabelle B

Antimykotische in-vivo-Wirksamkeit (oral) bei Mäusecandidose

| Wirkstoff | Wirkung |
|---|---|
| (A) (bekannt) | k. W. |
| (B) (bekannt) | k. W. |
| (C) (bekannt) | k. W. |
| (D) (bekannt) | k. W. |
| Verbindungen gemäß Herstellungs-Beispiel | |
| 1 | +++ |
| 3 | ++++ |
| 4 | +++++ |
| 5 | ++++ |
| 7 | +++++ |
| 8 | +++ |
| 9 | +++ |
| 10 | ++++ |

Fortsetzung

| Wirkstoff | Wirkung |
|---|---|
| Verbindungen gemäß Herstellungs-Beispiel | |
| 12 | ++++ |
| 13 | +++++ |
| 14 | +++++ |
| 15 | ++++ |
| 22 | +++++ |
| 23 | + |
| 24 | ++ |
| 25 | +++ |
| 26 | +++ |
| 27 | +++ |
| 28 | ++++ |
| 31 | ++++ |
| 32 | ++++ |
| 36 | ++++ |
| 37 | ++++ |
| 38 | +++ |
| 39 | ++ |
| 40 | ++ |

## Beispiel C

### Antimikrobielle in-vivo-Wirksamkeit (lokal) am Modell der experimentellen Meerschweinchen-Trichophytie

Versuchsbeschreibung:

Weiße Mäuse der Rasse Pribright-white wurden auf dem geschorenen, nicht skarifizierten Rücken mit einer Mikro- und Makro-Suspension von Trichophyton mentagrophytes infiziert.
Die infizierten Tiere wurden, beginnend mit dem 3. Tag p. i., 1 × täglich mit einer 15%igen Lösung der erfindungsgemäßen Präparate (in Dimethylsulfoxid : Glycerin = 1 : 4) lokal behandelt.

Ergebnis:

Bei unbehandelten Tieren entwickelte sich innerhalb 12 Tagen p. i. das typische Bild einer Dermatophytose mit Rötung, Schuppung und Haarausfall bis zum totalen Integument-Defekt an der Infektionsstelle.
In diesem Test zeigen z. B. die erfindungsgemäßen Verbindungen 5, 7, 8, 9, 10, 13, 14, 15, 25, 26, 27, 28, 31 und 32 Wirkung bis gute Wirkung.

Tabelle C

Antimykotische in-vivo-Wirksamkeit (lokal) am Modell der experimentellen Meerschweinchen-Trichophytie

| Wirkstoff | Wirkung |
|---|---|
| gemäß Herstellungs-Beispiel | |
| 1 | ++ |
| 5 | +++ |
| 7 | +++ |
| 8 | +++ |
| 9 | ++++ |
| 10 | ++++ |
| 13 | +++ |
| 14 | ++++ |
| 15 | ++++ |
| 22 | ++ |
| 25 | ++++ |
| 26 | +++ |
| 27 | +++ |
| 28 | ++++ |
| 31 | +++ |
| 32 | +++ |

| | | | | |
|---|---|---|---|---|
| +++++ | = | sehr gute Wirkung | = | keine Infektion am 12.–15. Tag p. i. |
| ++++ | = | gute Wirkung | = | geringe Rötung, vereinzelt Schuppen |
| +++ | = | Wirkung | = | Rötung, Schuppung, ohne Haarausfall |
| ++ | = | schwache Wirkung | = | Rötung, Schuppung, Haarausfall |
| + | = | Spur Wirkung | = | flächiger Haarausfall, entzündliche Hautreaktion |

Beispiel D

Formulierungen

1. Lösung:

| | |
|---|---|
| Wirkstoff gemäß Formel (I) | 10 g |
| Alkohol, rein (96%ig) | 300 g |
| Isopropylmyristat | 526 g |
| | 836 g |

2. Creme:

| | |
|---|---|
| Wirkstoff gemäß Formel (I) | 10 g |
| Arlacel 60® (Sorbitan-monostearat) | 20 g |
| Tween 60® (Polyoxyethylen(20)-sorbitanmonostearat) | 15 g |
| Walrat, künstlich (Mischung von Estern von gesättigten Fettsäuren $C_{14}$—$C_{18}$ und Fettalkoholen $C_{14}$—$C_{18}$) | 30 g |
| Lanette O® (Gemisch aus Cetyl-Alkohol und Stearyl-Alkohol) | 100 g |
| Entanol G® (2-Octyl-dodecanol) | 135 g |
| Benzylalkohol | 10 g |
| Wasser, entmineralisiert | 680 g |
| | 1000 g |

**Patentansprüche**

1. Antimykotisches Mittel, gekennzeichnet durch einen Gehalt an mindestens einem substituierten 1-Hydroxyalkyl-azolyl-Derivat der allgemeinen Formel

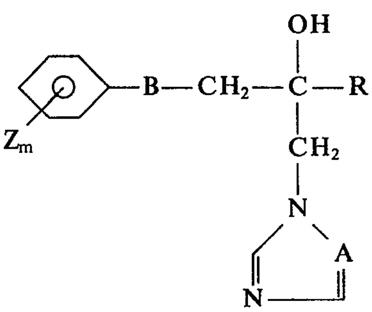

(I)

in welcher

A für ein Stickstoffatom oder die CH-Gruppe steht,
B für Sauerstoff, Schwefel oder die $CH_2$-Gruppe steht,
m für die Zahlen 0, 1, 2 oder 3 steht,
R für die Gruppierungen

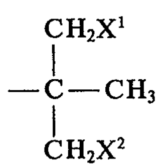 und 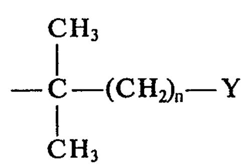

steht, wobei

$X^1$ für Wasserstoff oder Halogen steht;
$X^2$ für Halogen steht;
Y für Alkoxy, Alkylthio, Halogenalkoxy, Halogenalkylthio, Alkenyl mit bis zu 6 Kohlenstoffatomen, Alkoxycarbonyl mit 1 bis 4 Kohlenstoffatomen im Alkylteil, Cyano sowie für gegebenenfalls substituiertes Phenyl, Phenoxy, Phenylthio, Phenylalkoxy mit 1 bis 4 Kohlenstoffatomen im Alkylteil und Phenylalkylthio mit 1 bis 4 Kohlenstoffatomen im Alkylteil steht, wobei jeweils

als Phenylsubstituenten vorzugsweise genannt seien: Halogen, Alkyl mit 1 bis 4 Kohlenstoffatomen; Alkoxy und Alkylthio mit jeweils 1 bis 4 Kohlenstoffatomen; Halogenalkyl, Halogenalkoxy und Halogenalkylthio mit jeweils 1 bis 2 Kohlenstoffatomen und 1 bis 5 gleichen oder verschiedenen Halogenatomen, wie insbesondere Fluor- und Chloratomen, Cyclohexyl, Dialkylamino mit 1 bis 4 Kohlenstoffatomen in jedem Alkylteil, Nitro, Cyano, sowie Alkoxycarbonyl mit 1 bis 4 Kohlenstoffatomen im Alkylteil; und auch für Alkyl mit bis zu 6 Kohlenstoffatomen steht, wenn B für Schwefel steht,

n für die Zahlen 0, 1 oder 2 steht, und

Z für Halogen, Alkyl mit 1 bis 4 Kohlenstoffatomen, Cycloalkyl mit 5 bis 7 Kohlenstoffatomen, Alkoxy und Alkylthio mit jeweils 1 bis 4 Kohlenstoffatomen, Halogenalkyl und Halogenalkoxy sowie Halogenalkylthio mit jeweils 1 bis 2 Kohlenstoff- und 1 bis 5 gleichen oder verschiedenen Halogenatomen, wie insbesondere Fluor- und Chloratomen, sowie für jeweils gegebenenfalls durch Halogen und Alkyl mit 1 bis 2 Kohlenstoffatomen substituiertes Phenyl, Phenoxy und Phenylalkyl sowie Phenylalkoxy mit 1 bis 2 Kohlenstoffatomen im Alkylteil bzw. Alkoxyteil steht.

2. Antimykotisches Mittel nach Anspruch 1, gekennzeichnet durch einen Gehalt an mindestens einer Verbindung der Formel (I) in Anspruch 1, in der

R für die Gruppierungen

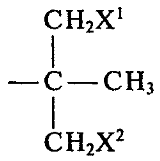 und 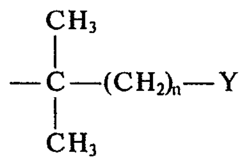

steht, wobei

$X^1$ für Wasserstoff, Fluor, Chlor oder Brom steht;

$X^2$ für Fluor, Chlor oder Brom steht;

Y für Methoxy, Ethoxy, Methylthio, Ethylthio, Trifluormethoxy, Trifluormethylthio, Vinyl, Methoxycarbonyl, Ethoxycarbonyl, Cyano sowie für gegebenenfalls substituiertes Phenyl, Phenoxy, Phenylthio, Phenylmethoxy und Phenylmethylthio steht, wobei jeweils als Phenylsubstituenten genannt seien: Fluor, Chlor, Methyl, Ethyl, Methoxy, Methylthio, Trifluormethyl, Trifluormethoxy, Trifluormethylthio, Dimethylamino, Methoxycarbonyl und Ethoxycarbonyl; und auch für Methyl, Ethyl und Propyl steht, wenn B für Schwefel steht,

n für die Zahlen 0, 1 oder 2 steht;

Z für Fluor, Chlor, Brom, Methyl, tert.-Butyl, Cyclohexyl, Methoxy, Methylthio, Trifluormethyl, Trifluormethoxy, Trifluormethylthio sowie für gegebenenfalls durch Fluor, Chlor und Methyl substituiertes Phenyl, Phenoxy, Benzyl oder Benzyloxy steht;

A, B und m für die in Anspruch 1 angegebenen Bedeutungen stehen.

3. Antimykotisches Mittel, gekennzeichnet durch einen Gehalt an 4-Fluor-2-(4-fluorphenoxymethyl)-3,3-dimethyl-1-(1,2,4-triazol-1-yl)-butan-2-ol.

4. Antimykotisches Mittel, gekennzeichnet durch einen Gehalt an 2-(4-Chlorphenylthiomethyl)-3,3-dimethyl-1-(1,2,4-triazol-1-yl)-butan-2-ol.

5. Antimykotisches Mittel, gekennzeichnet durch einen Gehalt an 4-(4-Biphenylyloxymethyl)-3,3-dimethyl-4-hydroxy-5-(1,2,4-triazol-1-yl)-1-penten.

6. Verfahren zur Herstellung eines antimykotischen Mittels, dadurch gekennzeichnet, daß man substituierte 1-Hydroxyalkyl-azolyl-Derivate gemäß Formel (I) in Anspruch 1 mit inerten, nichttoxischen, pharmazeutisch geeigneten Trägerstoffen vermischt.

**Claims**

1. Antimycotic agent, characterised in that it contains at least one substituted 1-hydroxyalkyl-azolyl derivative of the general formula

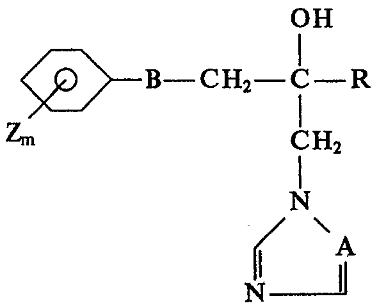

(I)

in which

A represents a nitrogen atom or the CH group,
B represents oxygen, sulphur or the $CH_2$ group,
m represents the numbers 0, 1, 2 or 3,
R represents the groupings

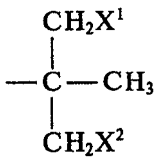 and 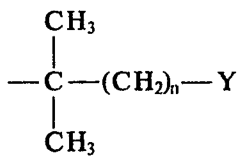

wherein

$X^1$ represents hydrogen or halogen;
$X^2$ represents halogen;
Y represents alkoxy, alkylthio, halogenoalkoxy, halogenoalkylthio, alkenyl with up to 6 carbon atoms, alkoxycarbonyl with 1 to 4 carbon atoms in the alkyl part, cyano and optionally substituted phenyl, phenoxy, phenylthio, phenylalkoxy with 1 to 4 carbon atoms in the alkyl part and phenylalkylthio with 1 to 4 carbon atoms in the alkyl part, the following being mentioned as preferable phenyl substituents in each case: halogen, alkyl with 1 to 4 carbon atoms; alkoxy and alkylthio with in each case 1 to 2 carbon atoms; halogenoalkyl, halogenoalkoxy and halogenoalkylthio with in each case 1 to 2 carbon atoms and 1 to 5 identical or different halogen atoms, such as, in particular, fluorine and chlorine atoms, cyclohexyl, dialkylamino with 1 to 4 carbon atoms in each alkyl part, nitro, cyano, and alkoxycarbonyl with 1 to 4 carbon atoms in the alkyl part; and also alkyl with up to 6 carbon atoms, when B represents sulphur,
n represents the numbers 0, 1 or 2, and
Z represents halogen, alkyl with 1 to 4 carbon atoms, cycloalkyl with 1 to 7 carbon atoms, alkoxy and alkylthio with in each case 1 to 4 carbon atoms, halogenoalkyl and halogenoalkoxy and halogenoalkylthio with in each case 1 to 2 carbon and 1 to 5 identical or different halogen atoms, such as, in particular, fluorine and chlorine atoms, and phenyl, phenoxy and phenylalkyl and phenylalkoxy with 1 to 2 carbon atoms in the alkyl part or in the alkoxy part, each of which is optionally substituted by halogen and alkyl with 1 to 2 carbon atoms.

2. Antimycotic agent according to Claim 1, characterised in that it contains at least one compound of the formula (I) in Claim 1, in which

R represents the groupings

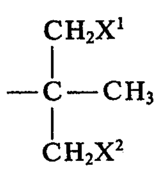 and 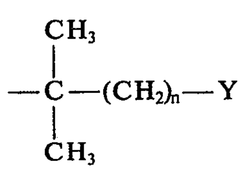

wherein

| | |
|---|---|
| $X^1$ | represents hydrogen, fluorine, chlorine or bromine; |
| $X^2$ | represents fluorine, chlorine or bromine; |
| Y | represents methoxy, ethoxy, methylthio, ethylthio, trifluoromethoxy, trifluoromethylthio, vinyl, methoxycarbonyl, cyano and optionally substituted phenyl, phenoxy, phenylthio, phenylmethoxy and phenylmethylthio, the following being mentioned as phenyl substituents in each case: fluorine, chlorine, methyl, ethyl, methoxy, methylthio, trifluoromethyl, trifluoromethoxy, trifluoromethylthio, dimethylamino, methoxycarbonyl and ethoxycarbonyl; and also methyl, ethyl and propyl, when B represents sulphur, |
| n | represents the numbers 0, 1 or 2; |
| Z | represents fluorine, chlorine, bromine, methyl, tert.-butyl, cyclohexyl, methoxy, methylthio, trifluormethyl, trifluoromethoxy, trifluoromethylthio and benzyloxy, benzyl, phenoxy or phenyl optionally substituted by fluorine, chlorine and methyl; and |
| A, B and m | represent the meanings given in Claim 1. |

3. Antimycotic agent, characterised in that it contains 4-fluoro-2-(4-fluorophenoxymethyl)-3,3-dimethyl-1-(1,2,4-tri azol-1-yl)-butan-2-ol.

4. Antimycotic agent, characterised in that it contains 2-(4-chlorophenylthiomethyl)-3,3-dimethyl-1-(1,2,4-triazol-1-yl)-butan-2-ol.

5. Antimycotic agent, characterised in that it contains 4-(4-biphenylyloxymethyl)-3,3-dimethyl-4-hydroxy-5-(1,2,4-triazol-1-yl)-pent-1-ene.

6. Process for the preparation of an antimycotic agent, characterised in that substituted 1-hydroxyalkyl-azolyl derivatives according to formula (I) in Claim 1 are mixed with inert, non-toxic, pharmaceutically suitable excipients.

## Revendications

1. Agent antimycotique, caractérisé en ce qu'il contient au moins un dérivé 1-hydroxyalkyl-azolylique substitué de formule générale

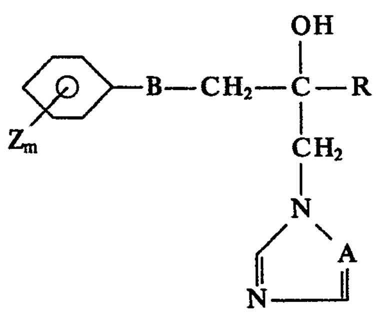 (I)

dans laquelle

| | |
|---|---|
| A | représente un atome d'azote ou un groupe CH, |
| B | représente l'oxygène, le soufre ou le groupe $CH_2$, |
| m | est égal à 0, 1, 2 ou 3, |
| R | représente les groupements |

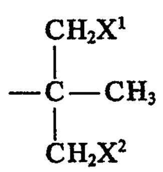 et 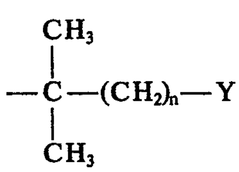

dans lesquels

| | |
|---|---|
| $X^1$ | représente l'hydrogène ou un halogène, |
| $X^2$ | représente un halogène, |
| Y | représente un groupe alcoxy, alkylthio, halogènoalcoxy, halogènoalkylthio, alcényle contenant jusqu'à 6 atomes de carbone, alcoxycarbonyle contenant 1 à 4 atomes de carbone dans la partie alkyle, cyano ou phényle, phénoxy, phénylthio, phénylalcoxy contenant 1 à 4 atomes de car- |

bone dans la partie alkyle et phénylalkylthio contenant 1 à 4 atomes de carbone dans la partie alkyle, éventuellement substitué, les substituants du noyau phényle étant de préférence: des halogènes, des groupes alkyle en $C_1$—$C_4$; alcoxy et alkylthio contenant chacun 1 à 2 atomes de carbone; halogènoalkyle, halogènoalcoxy et halogènoalkylthio contenant chacun 1 à 2 atomes de carbone et 1 à 5 atomes d'halogènes identiques ou différents, en particulier des atomes de fluor et de chlore, des groupes cyclohexyle, dialkylamino contenant 1 à 4 atomes de carbone dans chaque partie alkyle, nitro, cyano ou alcoxycarbonyle contenant 1 à 4 atomes de carbone dans la partie alkyle; et également un groupe alkyle contenant jusqu'à 6 atomes de carbone, lorsque B représente le soufre,

n est égal à 0, 1 ou 2,

Z représente un halogène, un groupe alkyle en $C_1$—$C_4$, cycloalkyle en $C_5$—$C_7$, alcoxy et alkylthio contenant chacun 1 à 4 atomes de carbone, halogènoalkyle et halogènoalcoxy ainsi que halogènoalkylthio contenant chacun 1 à 2 atomes de carbone et 1 à 5 atomes d'halogènes identiques ou différents, en particulier des atomes de fluor et de chlore, ainsi que phényle, phénoxy et phénylalkyle ainsi que phénylalcoxy contenant 1 à 2 atomes de carbone dans la partie alkyle ou dans la partie alcoxy, éventuellement substitué par des halogènes et des groupes alkyle en $C_1$—$C_2$.

2. Agent antimycotique selon la revendication 1, caractérisé en ce qu'il contient au moins un composé de formule (I) selon la revendication 1, dans laquelle

R représente les groupements

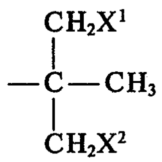 et 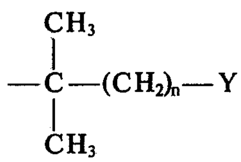

dans lesquels

$X^1$ représente l'hydrogène, le fluor, le chlore ou le brome;

$X^2$ représente le fluor, le chlore ou le brome;

Y représente un groupe méthoxy, méthylthio, éthylthio, trifluorométhoxy, trifluorométhylthio, vinyle méthoxycarbonyle, éthoxycarbonyle, cyano ainsi que phényle, phénoxy, phénylthio, phénylméthoxy et phénylméthylthio éventuellement substitué, les substituants du groupe phényle pouvant être dans chaque cas: le fluor, le chlore, des groupes méthyle, éthyle, méthoxy, méthylthio, trifluorométhyle, trifluorométhoxy, trifluorométhylthio, diméthylamino, méthoxycarbonyle et éthoxycarbonyle; et également méthyle, éthyle et propyle, lorsque B représente le soufre,

n est égal à 0, 1 ou 2;

Z représente le fluor, le chlore, le brome, un groupe méthyle, tert.-butyle, cyclohexyle, méthoxy, méthylthio, trifluorométhyle, trifluorométhoxy, trifluorométhylthio, ou phényle, phénoxy, benzyle ou benzyloxy éventuellement substitué par le fluor, le chlore ou des groupes méthyles;

A, B et m ont les significations indiquées dans la revendication 1.

3. Agent antimycotique, caractérisé en ce qu'il contient du 4-fluoro-2-(4-fluorophénoxyméthyl)-3,3-diméthyl-1-(1,2,4-triazole-1-yl)-butane-2-ol.

4. Agent antimycotique, caractérisé en ce qu'il contient du 2-(4-chlorophénylthiométhyl)-3,3-diméthyl-1-(1,2,4-tri azole-1-yl)-butane-2-ol.

5. Agent antimycotique, caractérisé en ce qu'il contient du 4-(4-biphénylyloxyméthyl)-3,3-diméthyl-4-hydroxy-5-(1,2,4-triazole-1-yl)-1-pentène.

6. Procédé de préparation d'un agent antimycotique, caractérisé en ce que l'on mélange des dérivés 1-hydroxyalkyl-azolyliques substitués de formule (I) de la revendication 1 avec des véhicules inertes et non toxiques appropriés à l'usage pharmaceutique.